# EUROPÄISCHE PATENTSCHRIFT

(11) **EP 1 001 951 B1**
(45) Veröffentlichungstag und Bekanntmachung des Hinweises auf die Patenterteilung: **25.09.2002**
(21) Anmeldenummer: 98936430.2
(22) Anmeldetag: 16.07.1998
(51) Int. Cl.: C07D 277/30, C07D 319/06, C07D 417/06, C07D 277/24, C07D 493/04

(54) **THIAZOLDERIVATE, VERFAHREN ZUR HERSTELLUNG UND VERWENDUNG**
THIAZOLE DERIVATIVES, METHOD FOR THEIR PRODUCTION AND USE
DERIVES DE THIAZOLE, LEUR PROCEDE DE PRODUCTION ET LEUR UTILISATION

(30) Priorität: 16.07.1997 DE 19731316
(43) Veröffentlichungstag der Anmeldung: 24.05.2000
(73) Patentinhaber: SCHERING AKTIENGESELLSCHAFT, 13353 Berlin (DE)
(72) Erfinder: MULZER, Johann, A-1090 Wien (AT); MANTOULIDIS, Andreas, A-1190 Wien (AT)
(86) Internationale Anmeldenummer: EP9804462
(87) Internationale Veröffentlichungsnummer: WO99003848

(56) Entgegenhaltungen:
- DE-A- 19 542 986
- DONGFANG MENG ET AL: "Studies toward a synthesis of epothilone A:Use of hydropyran templates for the management of acyclic stereochemical relationships" JOURNAL OF ORGANIC CHEMISTRY., Bd. 61, Nr. 23, 1996, Seiten 7998-7999, XP002035361 EASTON US in der Anmeldung erwähnt
- JOHANN MULZER ET AL: "Synthesis of the C(11)-C(20) segment of the cytotoxic macrolide epothilone B " TETRAHEDRON LETTERS., Bd. 38, Nr. 44, 3. November 1997, Seiten 7725-7728, XP002083207 OXFORD GB

## Beschreibung

Die Erfindung betrifft den in den Patentansprüchen gekennzeichneten Gegenstand, daß heißt Thiazolderivate, Verfahren zu ihrer Herstellung und ihre Verwendung zur Herstellung von Epothilon A, Epothilon B oder deren Derivaten.

Es ist bekannt, daß die Naturstoffe Epothilon A (R = H) und Epothilon B (R = Methyl) (Verbindung I, DE 195 42 986 Al, DE 41 38 042 C2) 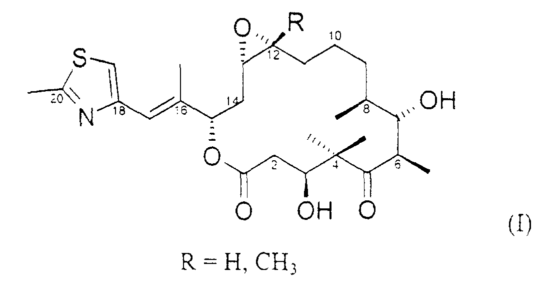 fungizid und cytotoxisch wirken. Nach Hinweisen für eine in vitro Aktivität gegen Brust- und Darmtumorzelllinien erscheint diese Verbindungsklasse in besonderem Maße interessant für die Entwicklung eines Arzneimittels, Verschiedene Arbeitsgruppen beschäftigen sich daher mit der Synthese dieser makrocyclischen Verbindungen. Die Arbeitsgruppen gehen von unterschiedlichen Bruchstücken des Makrocyclus aus, um die gewünschten Naturstoffe zu synthestisieren. Danishefsky et al plant die Synthese aus drei Bruchstücken C(1)-C(2) + C(3)-C(9) + C(10)-C(20). Bei dem C(10)-C(20)-Bruchstück handelt es sich um ein Thiazolderivat, das in einer 15-stufigen Synthese nicht diastereomerenrein erhalten werden konnte (JOC, 1996, 61, 7998-7999). Diastereomerenreinheit ist jedoch oft entscheidend für die Wirkung und Voraussetzung für die Herstellung eines Arzneimittels.

Es bestand daher die Aufgabe, geeignete Bruchstücke diastereomerenrein bereitzustellen. aus denen sich die makrocyclischen Verbindungen und deren Derivate synthetisieren lassen.

Es wurde nun gefunden, daß die Thiazolderivate der Formel II 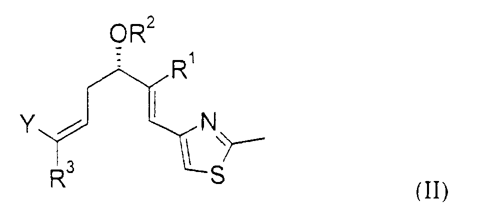 worin
- R¹: C₁-C₄-Alkyl,
- R²: eine beliebige chelatisierungsfähige Schutzgruppe,
- R³: Wasserstoff oder C₁-C₄-Alkyl
- Y: CO₂R⁴, CHO, CH=CH₂ oder CH₂R⁵,
wobei
R⁴ für C₁-C₄-Alkyl oder eine gegebenenfalls substituierte Benzylgruppe,
R⁵ für Halogen, Hydroxy, p-Toluolsulfonat oder -OSO₂B und
B für C₁-C₄-Alkyl oder C₁-C₄-Perfluoralkyl steht,
bedeutet,
sich diastereomerenrein herstellen lassen und geeignet sind für die Herstellung von Epothilon A und Epothilon B und deren Derivaten.

Unter C₁-C₄-Alkyl für R¹, R³, R⁴, und B sind Methyl, Ethyl, Propyl, Isopropyl. Butyl, Isobutyl und Tertiärbutyl zu verstehen.

Unter einer beliebigen chelatisierungsfähigen Schutzgruppe R² sind zum Beispiel Benzylreste wie z.B. Benzyl, p-Methoxybenzyl (PMB), Silylreste wie z.B. Trimethyl-silyl, 2-(Trimethylsilyl)ethoxymethyl (SEM), Tetrahydropyranyl, Methoxymethyl, Benzyloxymethoxymethyl, Benzoyl, Acetyl zu verstehen.

Die substituierte Benzylgruppe R⁴ kann z.B. p-Methoxybenzyl, 2,4-Dimethoxybenzyl oder ein durch andere elektronenschiebende Substituenten substituierter Benzylrest sein.

Mit Halogen sind Fluor, Chlor, Brom und Iod gemeint, wobei Brom und Iod bevorzugt sind.

Unter C₁-C₄-Perfluoralkyl sind geradkettige oder verzweigte vollständig fluorierte Alkylreste wie zum Beispiel CF₃, C₂F₅, C₃F₇, C₄F₉ zu verstehen.

Die Verbindungen II können nach dem in Schema I gezeigten Verfahren hergestellt werden, in dem die Synthese beispielhaft für Verbindung IIa mit R² = p-Methoxybenzyl, R³ = Methyl und Y = CO₂Et dargestellt ist.
Ausgehend von der natürlich vorkommenden (S)-Äpfelsäure (III) wird die α-Hydroxysäurefunktion mit Trifluoressigsäureanhydrid/Methanol (a) in den Mono-methylester überführt. Die noch verbliebene Säurefunktion wird dann mit Diboran in Tetrahydrofuran (b) zum Alkohol reduziert. Der so erhaltene (S)-(-)-Methyl-2,4-Dihydroxyester wird mit p-Methoxybenzyldimethylacetal mit Camphersulfonsäure in Toluol unter Rückfluß (c) in das cyclische Acetal (IV) überführt. Aus dem Methylester wird durch Reaktion mit einem Äquivalent Methyllithium in 2 Stunden bei -100°C (d) das Methylketon (V) erhalten. Umsetzung mit einer C₂-, C₃- oder C₄-metallorganischen Verbindung z.B. einer Grignardverbindung unter üblichen Reaktionsbedingungen führt zu den übrigen Resten R¹. Bei der Wittigreaktion (e) wird das 2-Methyl-4-thiazolylmethyltriphenylphosphoniumbromid, das in zwei Stufen aus 1,3-Dichlorpropanon zugänglich ist, zuerst mit Natriumhexamethyldisilazid bei -78°C in Tetrahydrofuran zusammengegeben bevor das Keton dazugegeben wird. Die Reaktion führt nach 1 Stunde und Erwärmen auf -40°C zu einem E/Z-Gemisch (E/Z = 3,6 : 1). Das E-Isomer (VI) ist durch einfache Flashchromatographie abzutrennen. Regioselektive Freisetzung der terminalen Hydroxygruppe durch reduktive Öffnung des Acetals mit 4 Äquivalenten Diisobutylaluminiumhydrid in Methylenchlorid in 4 Stunden bei -20°C (f) ergibt ein gut trennbares Gemisch ( 5,6 : 1 für das gewünschte Regioisomer) der Alkohole. Nach Trennung wird der Alkohol durch Swern-Oxidation in einer Stunde unter Aufwärmen von -78°C nach O°C (g) in den entsprechenden Aldehyd überführt, der sofort zur Wadsworth-Homer-Emmons-Kondensation unter Still's Bedingungen (h) mit Ethyl-2-Diethoxyphosphinylpropionat oder dem entsprechend dem gewünschten Rest R³ geeigneten Horner-Reagenz unter Zugabe von Kaliumhexamethyldisilazid, 18-Krone-6 bei -78°C für eine Stunde in Tetrahydrofuran umgesetzt wird. Es wird ein E/Z-Gemisch (E/Z = 6,2 : 1) der α,β-ungesättigten Ester erhalten, aus dem das Z-Isomer (IIa) in guter Ausbeute abgetrennt werden kann. Die Verwendung des Trifluorethylphosphonat-Derivates führt zu einer besseren Selektivität von 15:1.

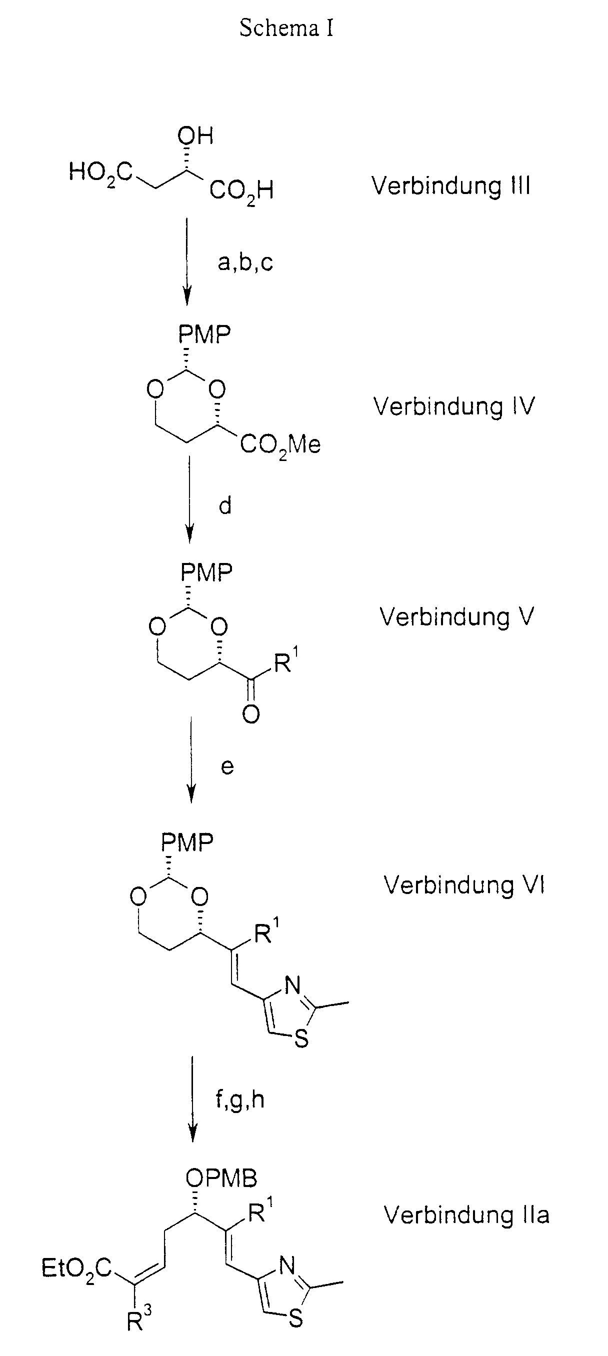

Die Verbindung der allgemeinen Formel IIa stellt einen zentralen Baustein für die Synthese von Epothilon-Derivaten und Epothilon selbst dar.

Die Esterfunktion in Position 11 kann in jede beliebige, für den späteren Ringschluß benötigte, Funktionalität überführt werden.

Derivatisierungen in 12-und 13-Position (Epothilon-Zählweise) sind aus der Doppelbindung möglich. So zum Beispiel die Überführung in das im Epothilon selbst vorhandene Epoxid durch Sharpless-Oxidation:
Dazu wird der Ester IIa mit 3 Äquivalenten Diisobutylaluminiumhydrid in Tetrahydrofuran bei -20°C (i) zum α,β-ungesättigten Alkohol reduziert und anschließend die Doppelbindung des Allylalkohols mit 4A Molekularsieb, Titantetraisopropylat, D-(-)-diisopropyltartrat. Tertiärbutylhydroperoxid in Methylenchlorid für 3 Stunden bei -30°C (k) diastereoselektiv epoxidiert.

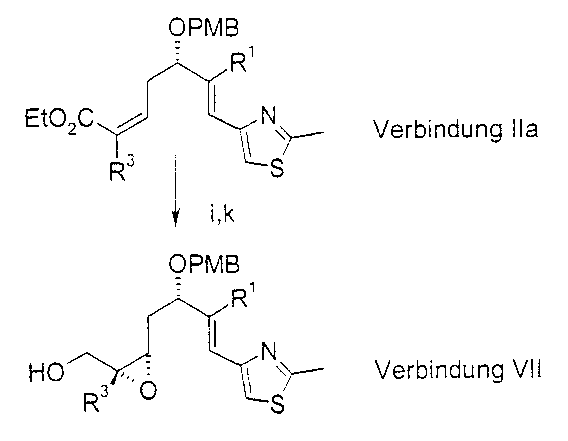

Auch die noch in geschützter Form vorliegende Hydroxyfunktion in 15-Position läßt Derivatisierungen an dieser Stelle zu oder ist unter literaturbekannten Bedingungen spaltbar.

Verbindungen mit Y = CHO können durch Dibal-Reduktion von Verbindung IIa in literaturbekannter Weise erhalten werden. Nachfolgende Wittigreaktion führt zu Verbindungen mit Y = CH=CH₂.

Die Verbindungen mit Y = CH₂R⁵ mit R⁵ = p-Toluolsulfonat, (C₁-C₄)alkylsulfonat, oder (C₁-C₄)perfluoralkylsulfonat können aus dem Alkohol (VII) erhalten werden.

Die Verbindungen mit Y = CH₂-Halogen lassen sich aus z.B. der Verbindung mit Y = CH₂-p-Toluolsulfonat oder Y = OH in üblicher Weise erhalten.

Im Gegensatz zu dem Verfahren von Danishefsky et al werden nur 10 Stufen für die Synthese bis zur Stufe des Epoxids benötigt und das Thiazolderivat der Formel Ha kann ebenso wie auch das Epoxid diastereomerenrein erhalten werden. Ein weiterer Vorteil besteht darin, daß das verwendete natürliche Ausgangsmaterial und die Reaktionen der Synthese eine Herstellung größerer Mengen erlauben.

Die Weiterverarbeitung der erfindungsgemäßen Verbindungen zu Epothilon A und B kann wie in der nachstehenden Reaktionssequenz angegeben erfolgen. Die Verbindung der allgemeinen Formel XI wird analog zu bekannten Verfahren durch Abspaltung der primären Schutzgruppe, Oxidation in Position 1, selektive Freisetzung der 15-Hydroxygruppe, wie sie beispielsweise von K.C. Nicolaou et al. In Nature, Vol. 387, 1997, S. 268-272 und J. Am, Chem. Soc. 1997, 119, S. 7960 - 7973 beschrieben sind. zu Epothilon B weiterverarbeitet: 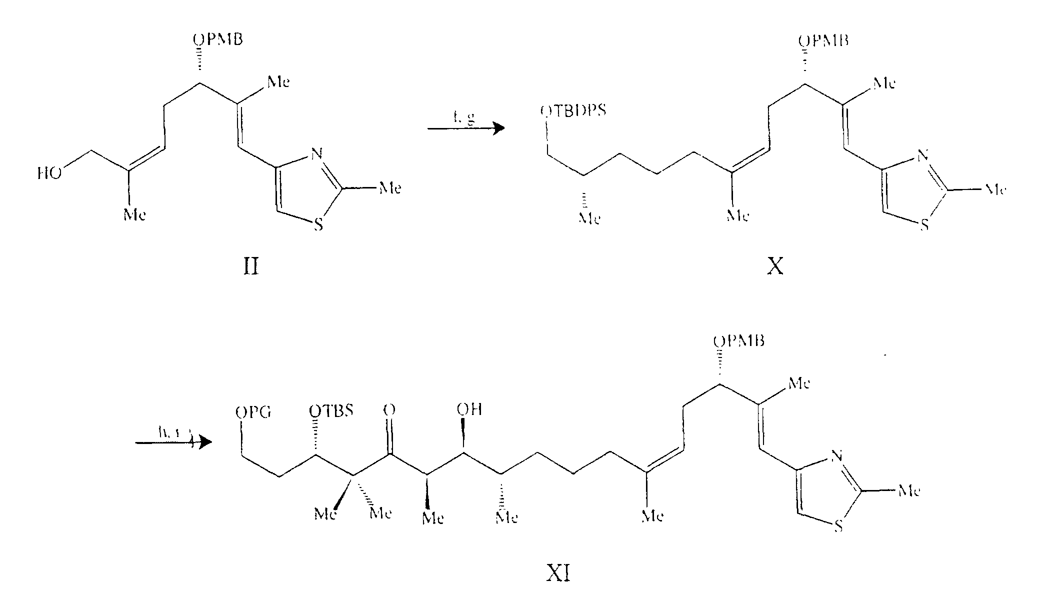 **f)** (i) Iodidbildung, (ii) Sulfonkupplung, 76,5%: **g)** Desulfonierung, 70%;
**h)** Desilylierung, 98%; **i)** Aldolreaktion.

Die nachfolgenden Beispiele dienen der näheren Erläuterung des Erfindungsgegenstandes, ohne ihn auf diese beschränken zu wollen.

### Präparative Methoden

Alle Umsetzungen metallorganischer Reagenzien und alle Reaktionen in absoluten Lösemitteln werden unter Luft- und Feuchtigkeitsausschluß durchgeführt. Die verwendeten Glasapparaturen werden vor Versuchsbeginn mehrmals im Ölpumpen-vakuum ausgeheizt und mit getrocknetem Argon der Firma Linde belüftet. Wenn nicht anders angegeben, werden sämtliche Reaktionsansätze magnetisch gerührt.

Methylenchlorid wird über eine basische Aluminiumoxidsäule der Aktivitätsstufe I (Woelm) getrocknet. Diethylether wird nach Vortrocknung auf einer basischen Aluminiumoxidsäule über eine 8:1 Natrium/Kalium-Legierung refluxiert bis zur stabilen Blaufärbung des Benzophenon-Indikators und vor der Verwendung frisch abdestilliert. Das Tetrahydrofuran (THF) wird über KOH vorgetrocknet, über eine mit basischem Aluminiumoxid beschickte Säule filtriert und anschließend über Kalium mit Triphenylmethan als Indikator destilliert.

Der Essigsäureethylester (EE) wird nach Vortrocknung über Calciumchlorid ebenso wie Hexan (Hex) vor der Verwendung zur Säulenchromatographie am Rotationsverdampfer abdestilliert.

### Chromatographische Verfahren

Sämtliche Reaktionen werden durch Dünnschichtchromatographie (DC) auf Kieselgel-60-Alufolien mit UV-Indikator F₂₅₄ der Firma Merck verfolgt. Als Laufmittel werden zumeist Lösemittelgemische aus Hexan (Hex) und Essigsäureethylester (EE) verwendet. Zum Sichtbarmachen nicht UV-aktiver Substanzen bewährt sich meist Anisaldehyd. Eisessig/Schwefelsäure (1:100:1) als Standard-Tauchreagenz.

Die präperative Säulenchromatographie wird an Kieselgel-60 der Firma Merck (0,04-0.063 mm, 230-400 mesh) durchgeführt, wobei als Eluens Lösemittelgemische aus Hexan (Hex) und Essigsäureethylester (EE) bzw. Diisopropylether dienen.

Im analytischen, wie auch im präperativen Maßstab werden die hochdruckflüssig-keitschromatographischen Trennungen (HPLC) auf Modulsystemen der Firmen Knauer (Pumpe 64, UV- und RI-Detektoren, Säulen und Schreiber), Waters/Millipore (Injek-tionssystem U6K9) und Milton-Roy (Integrator CI-10) durchgeführt. Für die analytische HPLC wird zumeist eine Knauer-Säule (4·250 mm) mit 5 µm Nucleosil und für die präperative HPLC eine Säule (16·250 mm, 32·250 mm bzw. 64·300 mm) mit 7 µ m oder 5 µm Nucleosil 50 verwendet.

### Färbereagenzien

Färbereagenz I (F I): 1 g Cer(IV)sulfat in 10 mL konz. Schwefelsäure und 90 mL Wasser liefert mit den meisten reduzierbaren Verbindungen intensiv blaue Farbreaktion beim Trocknen.

Färbereagenz II (F II): Eine 10%ige ethanolische Lösung von Molybdatophosphorsäure stellt ein weiteres Tauchreagenz zum Nachweis ungesättigter und reduzierbarer Verbindungen dar. Im Unterschied zum Färbereagenz I zeigt das Molydat-Färbereagenz, speziell auf einige Funktionalitäten ansprechend, ein breiteres Farbspektrum bei praktisch gleicher Zuverlässigkeit.

Färbereagenz III (F III): 1 mL Anisaldehyd in 100 mL Ethanol und 2 mL konz. Schwefelsäure stellt ein äußerst empfindliches Färbereagenz dar, daß zudem auch das wohl breiteste Farbspektrum zeigt.

Färbereagenz IV (F IV): Das Vanillin-Tauchbadreagenz ist ähnlich empfindlich, wie das Anisaldehyd-Färbereagenz und zeigt wie dieses ein nahezu breites Farbspektrum.

Färbereagenz V (F V): 1 g 2,4-Dinitrophenylhydrazin in 25 mL Ethanol, 8 mL Wasser und 5 mL konz. Schwefelsäure stellt ein hervorragendes, seletiv schon ohne Erwärmung auf Aldehyde und etwas langsamer auf Ketone ansprechendes, Tauchreagenz dar.

Färbereagenz VI (F VI): Eine 0.5%ige wässerige Lösung von Kaliumpermanganat zeigt durch Entfärbung oxidierbare Gruppen an, wobei ungesättigte, nicht aromatische Struktureinheiten spontan ohne Erwärmung reagieren.

### Spektroskopische Verfahren und allgemeine Analytik

### NMR-Spektroskopie

Die ¹H-NMR-Spektren werden mit einem AC 250, AM 270 oder AMX 500 Spektrometer der Firma Bruker mit den Substanzen als Lösung in deuterierten Lösemitteln und Tetramethylsilan als internem Standard aufgenommen. Die Auswertung der Spektren erfolgt nach den Regeln erster Ordnung. Ist eine auftretende Signalmultiplizität damit nicht zu erklären, erfolgt die Angabe des beobachteten Liniensatzes. Zur Bestimmung der Stereochemie wird die NOE-Spektroskopie (Nuclear Overhauser Effect) verwendet.

Zur Charakterisierung der Signale werden folgende Abkürzungen verwendet: s (Singulett), d (Dublett), dd (Doppeldublett), ddd (6-Liniensystem bei zwei gleichen Kopplungskonstanten bzw. ein 8-Liniensystem bei drei verschiedenen Kopplungs-konstanten), t (Triplett), q (Quartett), quint (Quintett), sext (Sextett), sept (Septett), m (Multiplett), mc (zentriertes Multiplett), br (breit) und v (verdecktes Signal).

Die ¹³C-NMR-Spektren werden mit einem AC 250 der Firma Bruker mit CDCl₃-Signal bei 77,0 ppm als internem Standard vermessen, wobei die Protonenresonanzen breitbandentkoppelt werden.

### Verwendete Abkürzungen

**abs.:** absolut, **Ar:** Aryl/Aromat, **ber.:** berechnet, **Brine:** kalt gesättigte Kochsalzlösung, **c:** Konzentration. **COSY:** korrelierte Spektroskopie (correlated spectroscopy), **DC:** Dünnschichtchromatographie. **DDQ:** Dichloro-dicyano-Quinon, **d.e.:** diastereomeric excess, **DIBAL:** Diisobutyl-aluminiumhydrid. **DMF:** N,N'-Dimethylformamid, **DMS:** Dimethylsulfid, **DMSO:** Dimethylsulfoxid, **ds:** Diastereoselektion, **EA:** Elementaranalyse, **e.e.:** enantiomeric excess. **EE:** Essigsäureethylester, **EI:** Elektronenstoßionisation, **eq:** Äquivalent(e), **eV:** Elektronenvolt, **FG:** functional group, **gef.:** gefunden, **ges.:** gesättigt(e), **h:** Stunde(n), **Hex:** n-Hexan, **HMDS:** Hexamethyldisilazid, **HPLC:** Hochdruckflüssigkeitschromatographie (high pressure liquid chromatographie, **Hünig Base:** N-Ethyl-diisopropylamin, **HRMS:** High Resolution Massenspektrometrie, **HV:** Hochvakuum, **iPrOH:** 2-Propanol, **IR:** Infrarotspektrometrie/Infrarotspektrum, **J:** Kopplungskonstante, **LDA:** Lithiumdiisopropylamin, **Lsg.:** Lösung, **Lsm.:** Lösemittel, **Me:** Methyl, **MeLi:** Methyllithium, **min:** Minute(n), **MS:** Massenspektrometrie/Massenspektren. **NMR:** Kernmagnetische Resonanz (Nuclear Magnetic Resonanz), **NOE:** Kern-Overhauser-Effekt (Nuclear Overhauser Effect), **PCC:** Pyridiniumchlorochromat, **PG:** Schutzgruppe (protection group), **Ph:** Phenyl, **ppm:** parts per million, **Rkt.:** Reaktion, **rt**: Retentionszeit, **RT:** Raumtemperatur (20-30 °C), **Std.:** Stunde(n), **TBAF:** Tetra-n-Butylammoniumfluorid, **TBDPS:** tert.-Butyldiphenyl-silyl-, **TBS:** tert.-Butyldimethylsilyl-, **tert./t:** tertiär, **TFA:** Trifluorethansäure, **TFAA:** Trifluorethansäureanhydrid. **TFMS:** Trifluormethansulfonsäure. **THF:** Tetrahydrofuran, **TMS:** Trimethylsilyl-, **u:** g mol⁻¹.

### Beispiel 1

### (2S,4S)-2-[4-Methoxyphenyl]-1,3-dioxan-4-carbonsäuremethylester

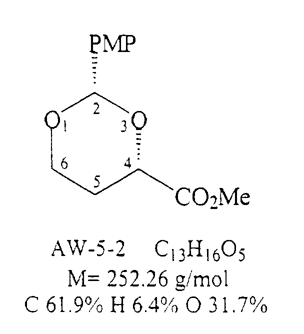

In einem ausgeheizten 250 ml Dreihalslöwenthalkolben werden 6.7 g (50 mmol) (S)-Äpfelsäure bei 0 °C unter Argon vorgelegt. Unter Rühren werden bei 0 °C 30 ml Trifluoressigsäureanhydrid über einen Tropftrichter sehr langsam zugegeben (Druckausgleich!). Nach vollständiger Zugabe wird das Eisbad entfernt und die Reaktionslösung noch 2 h bei Raumtemperatur gerührt.

Nun wird Trifluoressigsäure und überschüssiges Anhydrid zunächst im Wasserstrahlvakuum und anschließend an der Ölpumpe entfernt und der kristalline Rückstand bei 0 °C tropfenweise mit 4.5 ml Methanol versetzt (Druckausgleich, s.o.!) und nach Entfernung des Eisbades noch ca. 12 h gerührt.

Nach Einengung und Trocknung im Vakuum wird die kristalline Verbindung von (2S)-2-Hydroxy-butan-1,4-disäure-1-monomethylester in 70 ml abs. THF gelöst und bei 0 °C tropfenweise mit 100 ml einer IM Boran.THF-Komplex-Lsg. versetzt. 3 h nachgerührt und dann vorsichtig durch tropfenweise Zugabe von 60 ml Methanol die Reaktion abgebrochen. Nach Einengung am Rotationsverdampfer wird das zähe Öl zur Entfernung von Trimethylborat noch mehrfach mit Methanol versetzt und im Vakuum eingedampft. (Eventuell liegt die Dihydroxyverbindung im Gemisch mit Hydroxy-butyrolacton vor; das so gereinigte Rohprodukt wird direkt weiter umgesetzt).

In einem ausgeheizten 250 ml Dreihalslöwenthalkolben wird obiges Rohprodukt in 220 ml abs. Toluol mit 12.8 mL (65 mmol) Anisaldehyddimethylacetal vorgelegt, mit 1.16 g Campfersulfonsäure versetzt und über einen mit aktiviertem 4Å Molsieb gefüllten Soxhletextraktor unter Rückfluß 5 h gerührt. Nach Abkühlung der Lösung wird über eine mit Kieselgel beschickte Fritte filtriert, nachgewaschen mit Ether, mit ges. Natriumcarbonat-Lsg. ausgeschüttelt, über Magnesiumsulfat getrocknet, filtriert und eingeengt. Das Rohprodukt wird über eine 5:1-Hex/EE-Kieselgelsäule chromatographiert. Man erhalt 6.65 g (52.7%) des thermodynamischen Acetalproduktes als kristalline Verbindung.
^{**1**}**H-NMR** (400 MHz, CDCl₃): δ in ppm =
1.85 (dtd, *J*_{3a,3b}= 13.5 Hz, *J*_{3a,4a u. 2}=2.8 Hz, *J*_{3a,4b}= 1.5 Hz, 1H, 3a-H); 2.12 (dddd, *J*_{3b,3a}= 13.5 Hz, *J*_{3b,2}≅ *J*_{3b,4a}≅ 12.0 Hz, *J*_{3b,4b}= 5.0 Hz, 1H, 3b-H); 3.76+3.77 (s, 3H+3H, OC*H*₃+CO₂C*H*₃); 3.98 (ddd, *J*_{4a,3b}≅ *J*_{4a,4b}≅ 12.0 Hz, *J*_{4a,3a}= 2.5 Hz, 1H, 4a-H); 4.30 (ddd, *J*_{4b,4a}= 12.0 Hz, *J*_{4b,3b}= 5.0 Hz, *J*_{4b,3a}= 1.5 Hz, 1H, 4b-H); 4.49 (dd, *J*_{2,3b}= 12.0 Hz, *J*_{2,3a}= 2.8 Hz, 1H, 2-H); 5.47 (s, 1H, OC*H*ArO); 6.87 (dt, *J*_{ArH,ArH}= 8.5 Hz, *J*_{ArH,OC}_{*H*}_{ArO}= 2.0 Hz, 2H, ArH); 7.42 (d, *J*_{ArH,ArH}= 8.5 Hz, 2H, ArH).
^{**13**}**C-NMR** (100 MHz, CDCl₃): δ in ppm =
28.1 (C-3); 52.2 (C-6); 55.5 (C-11); 66.6 (C-4); 75.7 (C-2); 101.3 (C-5); 113.6 (C-9); 127.5 (C-8); 130.2 (C-7); 160.1 (C-10); 170.4 (C-1).
**IR** (Si-Film): ν in cm⁻¹ =
2961m; 2855m; 1730s; 1614m; 1519m; 1445m; 1375m; 1310s; 1251vs; 1207m: 1185m; 1137s; 1096s; 1070m; 1028vs; 993vs; 832s.
**MS** (EI, 70 eV, 30°C): m/e =
252 (98) [M⁺]; 251 (100) [M⁺-H]; 221 (14); 193 (86); 169 (16); 137 (88); 136 (98); 135 (98); 121 (28); 119 (34); 109 (42); 77 (53); 69 (58); 57 (25); 55 (31).
**Schmp.**: 78-80°C (aus Et₂O)

| | | | | |
|---|---|---|---|---|
| **C**_{**13**}**H**_{**16**}**O**_{**5**}**:** (M= 252.26 g·mol⁻¹) | **EA** | ber. | C: 61,90 % | H: 6,39 % |
| | | gef. | C: 61.67 % | H: 6.43 % |

### Beispiel 2

(2S,4S)-(2-[4-Methoxyphenyl]-1,3-dioxan-4-yl)-ethan-1-on

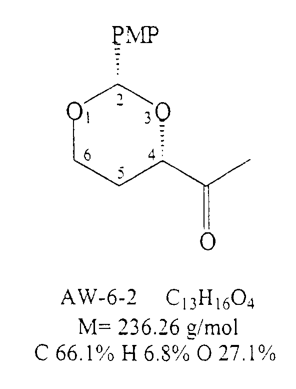

In einem 250 ml Dreihalsrundkolben werden 2.066 g (8.19 mmol) der aus Beispiel 1 erhaltenen Verbindung in ca. 80 ml abs. THF bei -100 °C tropfenweise mit 7.17 ml einer 1.6 M MeLi-Lsg. (1.4 eq) versetzt und 1-2 h nachgerührt.

Bei vollständigem Umsatz des Eduktes, wird das Kühlbad entfernt und zügig mit ca. 100 ml ges. NH₄Cl-Lsg. gequenscht und 1 h nachgerührt. Zur Aufarbeitung wird mit Ether verdünnt, die Phasen getrennt, die org. Phase mit Wasser, ges. NaHCO₃-Lsg., Wasser und Brine gewaschen und die wässerige Phase nochmals mit Ether extrahiert. Die vereinigten org. Phasen werden über Magnesiumsulfat getrocknet, filtriert und einrotiert, wobei das Produkt eventuell schon auskristallisiert (in diesem Fall kann zur Reinigung einfach mit kaltem Hexan gewaschen werden). Nach Chromatographie über eine 3:1-Hex/EE-Kieselgelsäule wurden 1.656 g (85.6%) erhalten.
^{**1**}**H-NMR** (400 MHz, CDCl₃): δ in ppm =
1.79 (dtd, *J*_{2a,2b}= 13.3 Hz, *J*_{2a,1a u. 3}= 2.9 Hz, *J*_{2a,1b} = 1.5 Hz, 1H, 2a-H); 1.90 (dddd, *J*_{2b,2a}= 13.3 Hz, *J*_{2b,2 u, 3}= 11.8 Hz, *J*_{2b,1b}= 4.9 Hz, 1H, 2b-H); 2.27 (s, 3H, COC*H*₃); 3.79 (s, 3H, OC*H*₃); 3.96 (td, *J*_{1a,1b}≅ *J*_{1a,2b}≅ 11.8 Hz, *J*_{1a,2a}= 2.5 Hz, 1H, 1a-H); 4.25(dd, *J*_{3,2b}= 11.3 Hz, *J*_{3,2a}= 3.0 Hz, 1H, 3-H); 4.29 (ddd, *J*_{1b,1a}= 11.3 Hz, *J*_{1b,2b}= 4.9 Hz. *J*_{1b,2a}= 1.0 Hz, 1H, 1b-H); 5.50 (s, 1H, OC*H*ArO); 6.89 (d, *J*_{ArH,ArH}= 8.8 Hz, 2H, ArH); 7.43 (d, *J*_{ArH,ArH}= 8.4 Hz, 2H, ArH).
^{**13**}**C-NMR** (100 MHz, CDCl₃): δ in ppm =
25.7 (C-5); 27.2 (C-2); 55.2 (C-11); 66.7 (C-1); 81.5 (C-2); 100.9 (C-6): 113.6 (C-9); 127.3 (C-8); 130.5 (C-7); 160.1 (C-10); 208.1 (C-1).
**IR** (Si-Film): ν in cm⁻¹ =
2999m; 2969s; 2931s; 2909m; 2871s; 2832m; 1710s; 1615m; 1590m; 1520s; 1464m; 1452m; 1429s; 1399m; 1359vs; 1328w; 1310m; 1296m; 1236vs; 1220m; 1207m; 1180s; 1119s; 1100s; 1069m; 1035vs; 1018vs; 992vs; 971vs; 948m: 833vs.
**MS** (EI, 70 eV, 30°C): m/e =
236 (88) [M⁺]; 235 (91); 221 (20); 194 (72); 193 (78); 163 (33); 153 (27); 137 (88); 136 (88); 135 (86); 121 (77); 109 (85); 100 (28); 92 (47); 84 (99); 83 (65); 77 (92); 65 (31); 63 (31); 57 (43); 55 (31); 43 (100).
**Schmp.:** 74-76°C

| | | | | |
|---|---|---|---|---|
| **C**_{**13**}**H**_{**16**}**O**_{**4**}**:** (M= 236.26 g·mol⁻¹) | **EA** | ber. | C: 66,09 % | H: 6.83 % |
| | | gef. | C: 66.34 % | H: 6.99 % |

### Beispiel 3

### (2'S,4'S,1E)-4-[2-(4-Methoxyphenyl-1,3-dioxan-4-yl)-prop-1-enyl]-2-methylthiazol

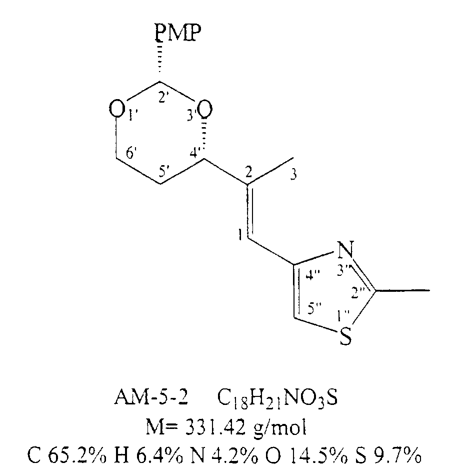

In einem 100 mL Dreihals-Löwenthalkolben werden 1.475 g (3.25 mmol; 1.3 eq) Wittigreagenz (2-Methyl-thiazol-4-yl-methyl-triphenylphosphoniumbromid); nach erneuter Trocknung im Ölpumpenvakuum mit 5 ml abs. THF suspendiert. Nach Abkühlung der Suspension auf -78 °C, wird mit einer Lösung von 715 mg (3.9 mmol; 1.2 eq) NaHMDS, gelöst in 5 ml abs. THF, durch langsame Zugabe deprotoniert und 15 min nachgerührt.

Nochmals direkt vor der Verwendung getrocknete 590 mg (2.5 mmol) der aus Beispiel 2 erhaltenen Verbindung, gelöst in 5 ml abs. THF. werden bei -78 °C langsam zugetropft, 5 min nachgerührt, anschließend das Kühlbad entfernt und auf Raumtemperatur erwärmen gelassen. Nach ca. 40 min wird die Reaktionslösung im Wasserbad auf 40-50 °C erwärmt und 1 h gerührt.

Zur Aufarbeitung wird durch Zugabe von ges. NH₄Cl-Lsg. gequenscht. die Phasen getrennt, die organische Phase über Magnesiumsulfat getrocknet, filtriert und einrotiert. Nach Chromatographie über eine 6:5:1-CH₂Cl₂/Hex/EE-Kieselgelsäule werden 171 mg Z-Olefin und 614 mg E-Olefin erhalten.

Die Olefinierungsprodukte werden somit in einer Ausbeute von 94.75% im Verhältnis von 1:3.6-*Z*:*E*-Olefin erhalten.
^{**1**}**H-NMR** (400 MHz, CDCl₃) (E-Olefin): δ in ppm =
1.67 (dtd, *J*_{2a,2b}= 13.3 Hz, *J*_{2a,1a u 3}= 2.5 Hz, *J*_{2a,1b}= 1.5 Hz, 1H, 2a-H); 2.02 (mc, 1H, 2b-H); 2.10 (d, *J*_{4, 5}= 1.0 Hz, 1H, 4-H); 2.69 (s, 3H. TAr-C*H*₃); 3.78 (s, 3H. OC*H*₃); 4.02 (td, *J*_{1a,1b}≅ *J*_{1a,2b}≅ 11.5 Hz, *J*_{1a,2a}= 2.5 Hz, 1H, 1a-H); 4.29 (ddd. *J*_{1b,1a}= 11.5 Hz, *J*_{1b,2b}= 5.0 Hz, *J*_{1b, 2a}= 1.5 Hz, 1H, 1b-H); 4.34 (mc, 1H, 3-H); 5.56 (s, 1H, OC*H*ArO); 6.63 (q, J_{5, 4}≅ 1.0 Hz, 1H, 5-H); 6.88 (mc, 2H, Ar-H); 6.97 (s, 1H, TAr-H); 7.44 (mc, 2H, Ar-H).
^{**13**}**C-NMR** (100 MHz, CDCl₃) (E-Olefin): δ in ppm =
15.1 (C-16); 19.2 (C-9); 30.2 (C-2); 55.3 (C-15); 67.1 (C-1); 81.7 (C-3); 101.1 (C-10);
113.5 (C-13); 115.7 (C-7); 118.9 (C-5); 127.5 (C-12); 131.3 (C-11); 139.1 (C-4); 152.8 (C-6); 159.9 (C-14); 164.4 (C-8).
**IR** (Si-Film): ν in cm⁻¹ =
3105w; 3057w; 2959m; 2925m; 2850m: 1658w; 1614s; 1517s; 1463m; 1442m: 1429m; 1394m; 1371m; 1302s; 1248vs; 1215w; 1172s; 1152w; 1118s; 1096s: 1062w; 1034s; 977w; 830m.
**MS** (EI, 70 eV, 40°C): m/e =
331 (41) [M⁺]; 279 (35); 247 (23); 231 (21); 195 (34); 178 (24); 167 (54); 164 (52); 149 (57); 140 (43); 139 (51); 136 (92); 135 (100); 119 (96); 97 (40); 94 (44); 91 (69); 77 (36); 69 (52); 57 (44); 55 (43); 43 (50).

| | | | | | |
|---|---|---|---|---|---|
| **C**_{**18**}**H**_{**21**}**NO**_{**3**}**S:** | **EA** | ber. | C: 65,23 % | H: 6,39 % | N: 4.22 % |
| (M= 331.42 g·mol⁻¹) | | gef. | C: 65.37 % | H: 6.41 % | N: 4.40 % |

### Beispiel 4

### (3S,4E)-3-[(4-Methoxyphenyl)methoxy]-4-methyl-5-(2-methylthiazol-4-yl)pent-4-enol

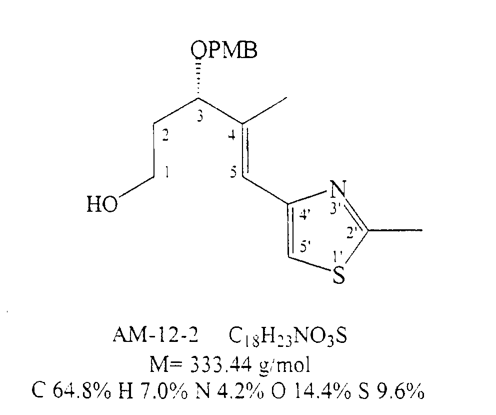

In 30 ml abs. CH₂Cl₂ werden 662 mg (2 mmol) der aus Beispiel 3 erhaltenen Verbindung bei -20 °C tropfenweise mit 8 ml einer IM DIBAL-Lsg. (4 eq) versetzt und ca. 5 h gerührt. Zum Reaktionsabbruch wird mit 1 ml MeOH gequenscht und anschließend langsam gesättigte NaK-Tartrat-Lsg. (30 ml) hinzugegeben. Die Lsg. wird über Nacht gerührt, wobei sich zwei klare Phasen gebildet haben. Die Phasen werden getrennt, die wässerige Phase noch zweimal mit CH₂Cl₂ extrahiert und die vereinigten org. Phasen mit ges. NH₄Cl-Lsg. gewaschen. Nach Trocknung über MgSO₄ wird filtriert und im Vakuum eingeengt.

Chromatographie über eine 2:1-Hex/EE-Kieselgelsäule erbrachte 594 mg (89.1%) Gesamtausbeute im Verhältnis 15:85 ((89 mg); (505 mg)).
^{**1**}**H-NMR** (400 MHz, CDCl₃) : δ in ppm =
1.68 (dq, *J*_{2a,2b}= 14.3 Hz, *J*_{2a, l's u, 3} = 4.9 Hz, 1H, 2a-H); 1.94 (mc, 1H, 2b-H); 1.99 (s, 3H, 4-H); 2.37 (br s, 1H, 1-OH); 2.66 (s, 3H, TAr-C*H*₃); 3.68 (br mc, 2H, 1-H); 3.73 (s, 3H, OC*H*₃); 3.99 (dd, *J*_{3.2a}*=* 8.9 Hz, *J*_{3,2b}= 3.9 Hz, 1H, 3-H); 4.18+4.42 (je d, *J*= 11.3 Hz, 2H, OC*H*₂Ar); 6.48 (s, 1H, 5-H); 6.80 (mc, 2H, Ar-H); 6.93 (s, 1H, TAr-H); 7.18 (mc, 2H, Ar-H).
^{**13**}**C-NMR** (100 MHz, CDCl₃) : δ in ppm =
13.6 (C-16); 19.2 (C-9); 36.7 (C-2); 55.2 (C-15); 61.1 (C-1); 69.9 (C-3); 84.3 (C-10); 113.9 (C-13); 115.9 (C-7); 121.1 (C-5); 129.4 (C-12); 130.2 (C-11): 139.1 (C-4); 152.6 (C-6); 159.2 (C-14); 164.7 (C-8).
**IR** (Si-Film): ν in cm⁻¹ =
3396br; 2926m; 2856w; 2835w; 1612m; 1586w; 1514vs; 1464m; 1453m; 1442m; 1302m; 1248vs; 1181m; 1173m; 1060m; 1035s; 821m.
**MS** (EI, 70 eV, 40°C): m/e =
333 (9) [M⁺]; 281 (14); 231 (14); 212 (40); 197 (51); 164 (30); 135 (22); 122 (40); 121 (100); 113 (31); 97 (23); 91 (39); 77 (37); 69 (38).

| | | | | | |
|---|---|---|---|---|---|
| **C**_{**18**}**H**_{**23**}**NO**_{**3**}**S:** | **EA** | ber. | C: 64,84 % | H: 6,95 % | N: 4.20 % |
| (M= 333.44 g·mol⁻¹) | | gef. | C: 65.08 % | H: 7.00 % | N: 4.14 % |

### Beispiel 5

### (5S,2Z,6E)-2,6-Dimethyl-5-[(4-ethoxyphenyl)methoxy]-7-(2-methylthiazol-4-yl)hepta-2,6-diensäure-ethylester

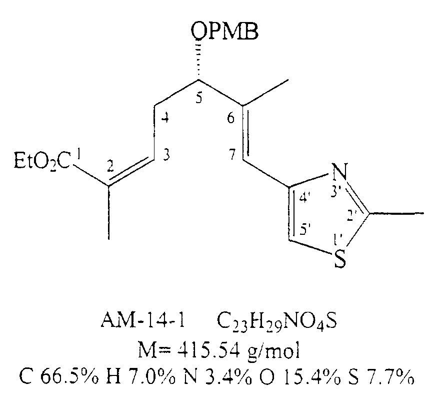

In 30 ml abs. CH₂Cl₂ werden 102 µL Oxalylchlorid (1,1 eq) vorgelegt und nach Einkühlung auf -78°C unter Argon langsam mit 187 µL DMSO (2,5 eq) versetzt und 10 min nachgerührt. (Trübung)

Bei -78 °C werden 354 mg (1,062 mmol)der aus Beispiel 4 erhaltenen Verbindung, gelöst in 5 ml abs. CH₂Cl₂, langsam zugegeben und 10 min nachgerührt. Anschließend wird ca. 1 ml (>5 eq) Hünigbase zugegeben, 15 min nachgerührt und dann das Kühlbad entfernt. (Wieder klare Lsg.). Die Reaktionslösung wird mit 40 ml einer 1:1-Hex/EE-Lsg. verdünnt und mit Eiswasser gequenscht. Die Phasen werden getrennt, die wässerige Phase noch zweimal mit Ether extrahiert und die vereinigten organischen Phasen über Magnesiumsulfat getrocknet, über eine kurze Kieselgelfritte filtriert, im Vakuum eingeengt und an der Ölpumpe getrocknet. Der Rohaldehyd wird ohne weitere Aufreinigung direkt für die nachfolgende Umsetzung verwendet.

In 25 ml abs. THF werden 303,5 mg 2-Phosphonopropionsäure-triethylester (1,2 eq) und 842 mg 18-Krone-6 (3 eq) bei -78 °C vorgelegt. Bei dieser Temperatur wird durch langsame Zugabe von 239 mg KHMDS (1,15 eq), gelöst in ca. 5 ml abs. THF, deprotoniert und 10 min nachgerührt. Anschließend wird der Rohaldehyd, gelöst in ca. 10 ml abs. THF, langsam zugegeben. DC-Kontrolle nach ca. 30 min zeigte bereits vollständigen Umsatz, so dass das Kühlbad entfernt und die Reaktion durch Zugabe von ges. NH₄Cl-Lsg. gequenscht wurde.

Nach Phasentrennung wird mit ges. NaHCO₃-Lsg, gewaschen, die wässerigen Phasen noch zweimal mit Ether extrahiert und die vereinigten organischen Phasen über Magnesiumsulfat getrocknet. Nach Filtration der organischen Phasen über kurze Kieselgelfritte wird am Rotationsverdampfer eingeengt. Chromatographie über ein 3:1-Hex/EE-Kieselgelvorsäule erbrachte 377 mg (85,46%) Isomerengemisch im Verhältnis von ca. 6,2:1 . Zur Trennung der Doppelbindungsisomere emphielt sich eine Chromatographie über eine 7:1-Hex/EE-Kieselgelsäule oder eine Reinigung auf der präperativen HPLC.

(Mitlerweile wurde auch die Verwendung des Trifluorethyl-Phosphonat-Derivates untersucht, die eine Selektivität von 15:1 erbrachte).
^{**1**}**H-NMR** (400 MHz, CDCl₃) (Z-Isomer): δ in ppm =
1.28 (t, *J*= 7.5 Hz, 3H, -CO₂CH₂C*H*₃); 1.88 (d, *J*_{*2, 3*}= 1.5 Hz, 3H, 2-H); 2.04 (d, *J*₆ ₇= 1.0 Hz, 3H, 6-H); 2.73 (s, 3H, TAr-C*H*₃); 2.82 (mc, 2H, 4-H's); 3.80 (s, 3H, OC*H*₃); 3.88 (t, *J*_{5.4a u. 4b}= 7.0 Hz, 1H, 5-H); 4.17 (q, *J=* 7.0 Hz, 2H, - CO₂C*H*₂CH₃); 4.24+4.49 (je d, *J=* 11.5 Hz, 2H, OC*H*₂Ar); 5.96 (tq, *J*_{3,4a u. 4b}= 6.9 Hz, *J*_{*3, 2*}= 1.5 Hz, 1H, 3-H); 6.54 (s, 1H, 6-H); 6.87 (mc, 2H, Ar-H); 6.99 (s, 1H, TAr-H); 7.25 (mc, 2H, Ar-H).
^{**13**}**C-NMR** (100 MHz, CDCl₃): δ in ppm =
13.4 (C-20); 14.3 (C-13); 19.2 (C-11); 20.7 (C-21); 34.4 (C-4); 55.3 (C-19); 60.1 (C-12); 69.8 (C-14); 84.3 (C-5); 113.7 (C-17); 115.8 (C-9); 121.4 (C-7); 128.4 (C-2); 129.4 (C-16); 130.7 (C-15); 138.8 (C-3); 139.1 (C-6); 152.7 (C-8): 159.1 (C-18); 164.5 (C-10); 167.9 (C-1).
**MS** (EI, 70 eV, 110°C): m/e =
415 (8) [M⁺]; 371 (13) [M⁺-OEt]; 294 (20); 289 (40); 288 (100); 248 (26); 231 (18); 204 (18); 164 (29); 138 (30); 122 (96); 121 (92); 113 (28); 97 (61); 91 (39); 78 (50); 77 (71); 69 (40); 53 (45); 43 (37).

| | | | | | |
|---|---|---|---|---|---|
| **C**_{**23**}**H**_{**29**}**NO**_{**4**}**S:** | **EA**: | ber.: | C: 66,48 % | H: 7,03 % | N: 3.37 % |
| (M= 415.54 g·mol⁻¹) | | gef.: | C: 65.91 % | H: 6.77 % | N: 3.29 % |

### Beispiel 6

### (5S,2Z,6E)-2,6-Dimethyl-5-[(4-methoxyphenyl)methoxy]-7-(2-methyl-thiazol-4-yl)hepta-2,6-dienol

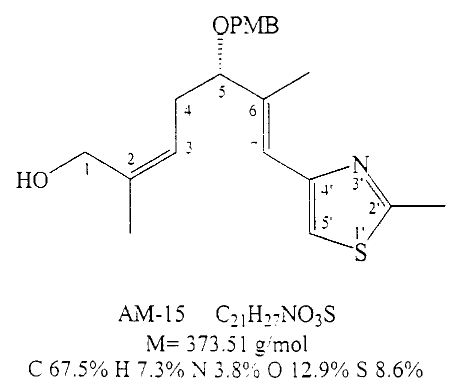

In 100 ml abs. THF werden bei -20 °C 417 mg (1,0035 mmol) der aus Beispiel 5 erhaltenen Verbindung vorgelegt und dann tropfenweise mit 3 ml einer 1M-DIBAL in Heptan Lösung versetzt. Nach 3 h wurde zur Vervollständigung des Reaktionsumsatzes noch 1 ml der DIBAL-Lsg. nachgegeben und nochmals 30 min bei -20 °C nachgerührt.

Zum Reaktionsabbruch wurde mit 1 ml MeOH gequenscht und nach Verdünnung mit 50 ml Diethylether werden 100 ml halbkonz. NaK-Tartrat-Lsg. zugegeben. Nach ca. 2-3 h kräftigen Rührens bei RT werden die Phasen getrennt, die wässerige Phase noch zweimal mit Ether extrahiert und die vereinigten organischen Phasen über Magnesiumsulfat getrocknet, filtriert und im Vakuum eingeengt. Chromatographische Reinigung über eine 1:1-Hex/EE-Kieselgelsäule erbrachte 272 mg (72,56%) Vinylalkohol.
^{**1**}**H-NMR** (400 MHz, CDCl₃): δ in ppm =
1.79 (s, 3H, 2-H); 2.03 (d, *J*_{6, 7}= 1.0 Hz, 3H, 6-H); 2.21 (mc, 1H, 4a-H); 2.47 (br. 1H, 1-OH); 2.52 (dt, *J*_{4b, 4a}= 14.3 Hz, *J*_{*4b, 3 u. 5*}*=* 8.4 Hz, 1H, 4b-H); 2.70 (s, 3H. TAr-C*H*₃); 3.75 (dd, *J*_{5, 4a}= 8.4 Hz, *J*_{5, 4b}= 4.4 Hz, 1H, 5-H); 3.77 (s, 3H, OC*H*₃); 3.84+4.13 (je br d, *J=* 11.8 Hz, 2H, 1-H's); 4.20+4.46 (je d, *J=* 11.3 Hz. 2H. OC*H*₂Ar); 5.26 (t, *J*_{3,4a u. 4b}= 8.0 Hz, 1H, 3-H); 6.49 (s, 1H, 7-H); 6.84 (mc, 2H, Ar-H); 6.97 (s, 1H, TAr-H); 7.20 (mc, 2H, Ar-H).
^{**13**}**C-NMR** (100 MHz, CDCl₃): δ in ppm =
13.8 (C-18); 19.2 (C-11); 22.2 (C-19); 34.0 (C-4); 55.2 (C-17); 61.3 (C-1); 70.0 (C-12); 83.7 (C-5); 113.7 (C-15); 115.8 (C-9); 121.1 (C-7); 123.8 (C-3); 129.6 (C-14); 129.9 (C-13); 138.2 (C-2); 139.4 (C-6); 152.6 (C-8); 159.2 (C-16); 164.7 (C-10).
**MS** (EI, 70 eV, 50°C): m/e =
373 (9) [M⁺]; 357 (8); 307 (11); 289 (27); 288 (96); 219 (19); 197 (17); 167 (39); 164 (28); 149 (33); 138 (41); 122 (100); 121 (92); 119 (34); 109 (27); 97 (52); 91 (81); 78 (39); 77 (56); 69 (36); 43 (56).

### Beispiel 7

### (5 S,2Z,6E)-2,6-Dimethyl-2,3-epoxy-5-[(4-methoxyphenyl)-methoxy]-7-(2-methylthiazol-4-yl)hept-6-enol

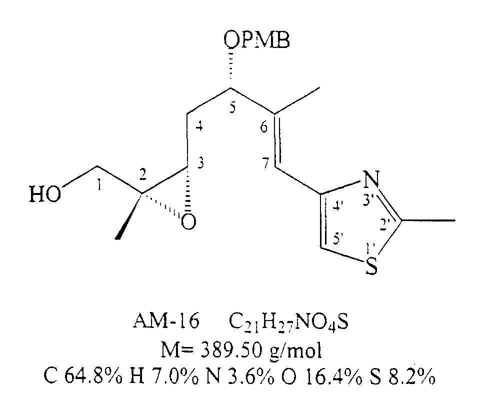

Zu einer Suspension von ca. 80 mg aktiviertem, zerstoßenem 3Å Molsieb in 2 ml abs. CH₂Cl₂ werden bei -15 °C 20,5 mg (0,0874 mmol) D-(-)-Diisopropyl-Tartrat und 21,7 µ 1 (7,28 µmol) Titanisopropoxid zugegeben.

Bei -30 °C werden 199 µl einer ca. 5,5M tert.-Butylhydroperoxid-Lsg. in Nonan langsam zugetropft, 10 min nachgerührt. Anschließend wird die resultierende Reagenzlösung bei -30 °C tropfenweise mit 265 mg (0,7095 mmol) der aus Beispiel 5 erhaltenen Verbindung , gelöst in ca. 1 ml abs. CH₂Cl₂, versetzt und 3 d gerührt.

Zur Aufarbeitung der Reaktion wird zunächst mit 15 ml CH₂Cl₂ verdünnt. 1 ml Wasser zugegeben und 30 min nachgerührt. Anschließend werden 1 ml (Brine/3N NaOH=1:1) zugegeben und wiederum 30 min kräftig nachgerührt. Nach Phasentrennung, zweimaliger Extraktion der wässerigen Phase mit CH₂Cl₂, Trocknung der vereinigten organischen Phasen über Magnesiumsulfat und Filtration über eine kurze Celite-Fritte wird im Vakuum eingeengt. Chromatographie über eine 1:1-Hex/EE-Kieselgelsäule erbrachte 235 mg (215 mg direkt und 20 mg ex ¹³C-Daten in der Mischfraktion) (85,04%) und noch 40 mg Gemischrest.
^{**1**}**H-NMR** (400 MHz, CDCl₃): δ in ppm =
1.40 (s, 3H, 2-H); 1.76 (ddd, ²*J*_{4a, 4b}= 15.3 Hz, *J*_{4a, 5}= 10.8 Hz, *J*_{4a, 3}= 9.9 Hz, 1H, 4a-H); 2.01 (ddd, ²*J*_{4b, 4a}= 14.8 Hz, *J*_{4b, 3}= 3.4 Hz, *J*_{4b, 5}= 2.5 Hz, 1H, 4b-H); 2.04 (d, ⁴*J*_{6, 7}= 1.0 Hz, 3H, 6-H); 2.71 (s, 3H, TAr-C*H*₃); 2.76 (dd, *J*_{3, 4a}= 9.9 Hz, *J*_{3, 4b}= 3.5 Hz, 1H, 3-H); 3.29 (dd, *J*_{1-OH, 1}= 10.8 Hz, *J*_{1-OH, 1}= 2.0 Hz, 1H, 1-OH); 3.45 (dd, ²*J*_{1a, 1b}=11.8 Hz, *J*_{1a, 1-OH}= 2.0 Hz, 1H, 1a-H); 3.61 (t br, ²*J*_{1b, 1a}=11.3 Hz, 1H, 1b-H); 3.78 (s, 3H, OC*H*₃); 3.99 (dd, *J*_{5, 4a}= 10.8 Hz, *J*_{5, 4b}= 2.5 Hz, 1H, 5-H); 4.22+4.51 (je d, ²*J*= 11.5 Hz, 2H, OC*H*₂Ar); 6.49 (d, ⁴*J*= 1.0 Hz, 1H, 7-H); 6.86 (mc, 2H, Ar-H); 7.00 (s, 1H, TAr-H); 7.22 (mc, 2H, Ar-H).
^{**13**}**C-NMR** (100 MHz, CDCl₃): δ in ppm =
13.4 (C-18); 19.2 (C-11); 20.4 (C-19); 33.7 (C-4); 55.2 (C-17); 60.5 (C-1); 62.1 (C-3); 64.2 (C-2); 70.0 (C-12); 81.3 (C-5); 113.9 (C-15); 116.4 (C-9); 121.7 (C-7); 129.0 (C-14); 131.1 (C-13); 138.1 (C-6); 152.3 (C-8); 159.5 (C-16); 164.9 (C-10).

## Patentansprüche

1. Verbindungen der allgemeinen Formel II 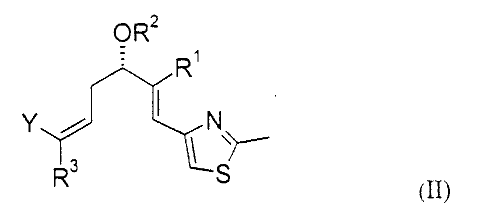 worin
R¹ C₁-C₄-Alkyl,
R² eine beliebige chelatisierungsfähige Schutzgruppe,
R³ Wasserstoff oder C₁-C₄-Alkyl
Y CO₂R⁴, CHO, CH=CH₂ oder CH₂R⁵,
wobei
R⁴ für C₁-C₄-Alkyl oder eine gegebenenfalls substituierte Benzylgruppe.
R⁵ für Halogen, Hydroxy, p-Toluolsulfonat oder -OSO₂B und
B für C₁-C₄-Alkyl oder C₁-C₄-Perfluoralkyl steht,
bedeutet.

2. Verbindungen der allgemeinen Formel II 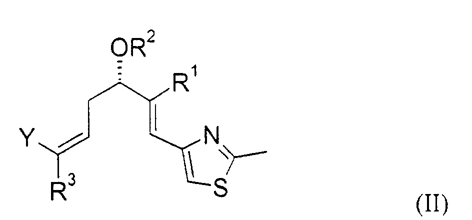 worin
R¹ C₁-C₄-Alkyl,
R² p-Methoxybenzyl
R³ Methyl und
Y CO₂R⁴
mit R⁴ C₁-C₄-Alkyl
bedeutet.

3. Verbindung der Formel IV 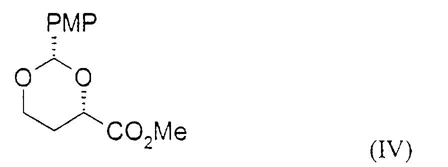 worin PMP p-Methoxyphenyl bedeutet.

4. Verbindungen der Formel V 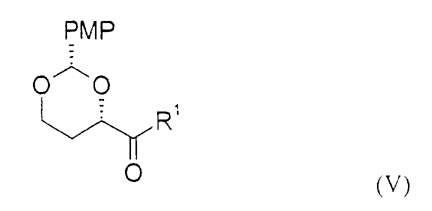 worin
R¹ C₁-C₄-Alkyl und
PMP p-Methoxyphenyl bedeutet.

5. Verbindungen der Formel VI 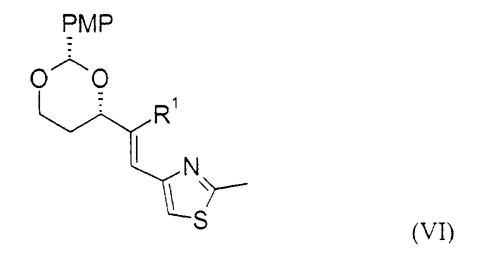 worin
R¹ C₁-C₄-Alkyl und
PMP P-Methoxyphenyl bedeutet.

6. Verbindungen der Formel IIa 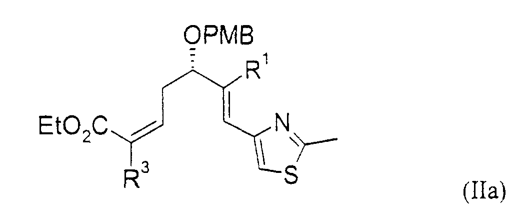 worin
R¹ C₁-C₄-Alkyl
PMB p-Methoxybenzyl bedeutet.
R³ Wasserstoff oder C₁-C₄-Alkyl

7. Verbindungen der Formel VII 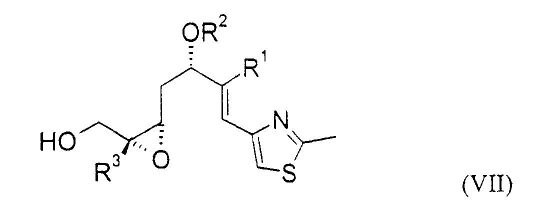 worin
R¹ C₁-C₄-Alkyl
R² eine chelatisierungsfähige Schutzgruppe darstellt.
R³ Wasserstoff oder C₁-C₄-Alkyl

8. Verfahren zur Herstellung der Verbindung der allgemeinen Formel IIa 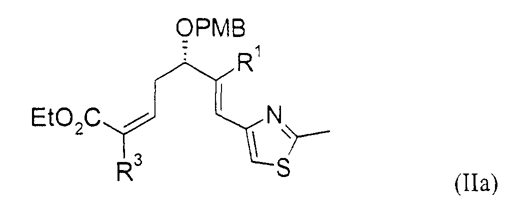 **dadurch gekennzeichnet, daß**
in einem Schritt 1
von (S)-Äpfelsäure (III) die α-Hydroxysäurefunktion mit Trifluoressigsäure/Methanol (a) in den Methylester überführt wird, die noch vorhandene Säurefunktion mit Diboran in Tetrahydrofuran (b) zum Alkohol reduziert wird und der so erhaltene (S)-(-)-Methyl-2,4-Dihydroxyester mit p-Methoxybenzyldimethylacetal (c) in das cyclische Acetal (IV) überführt wird, 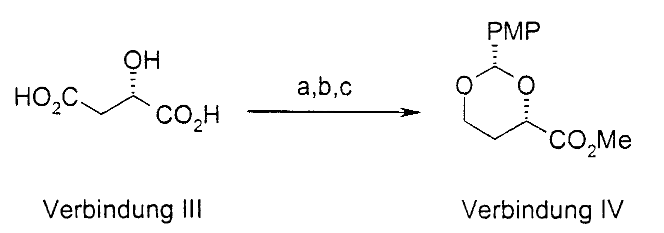 in einem Schritt II
der Methylester mit einer C₁-C₄-Alkyl-metallorganischen Verbindung (d) in das entsprechende Alkylketon (V) überführt wird, 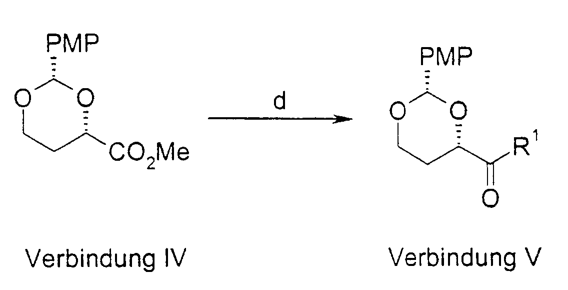 in einem Schritt III
das (C₁-C₄)-Alkylketon (V) in einer Wittigreaktion mit dem Thiazolylphosphoniumsalz (e) umgesetzt und das E-Isomere (VI) abgetrennt wird und 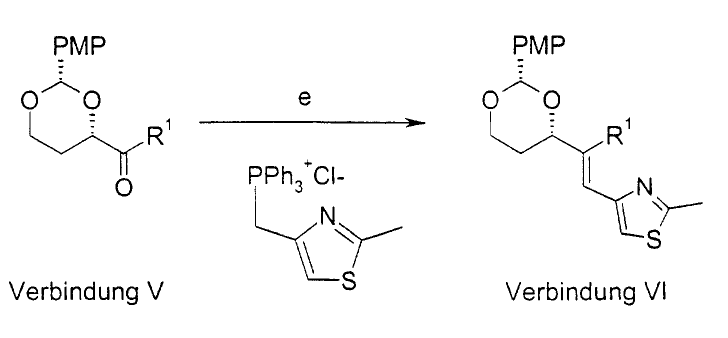 in einem Schritt IV
das E-Isomere (VI) durch Reaktion mit Diisobutylaluminiumhydrid (f), Swern-Oxidation (g) und Wadsworth-Horner-Emmons-Kondensation (h) mit Ethyl-2-Diethoxyphosphinylpropionat oder einem für R³ entsprechenden Horner-Reagenz und Reinigung vom E-Isomeren in den Z- α,β-ungesättigten Ester (IIa) überführt wird.

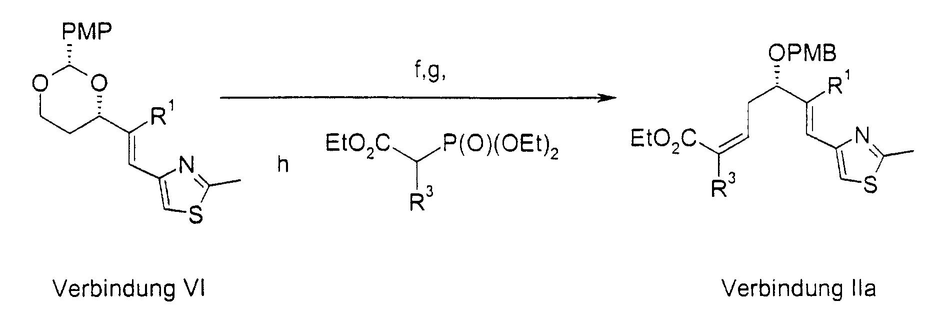

9. Verbindungen der allgemeinen Formel VIIa 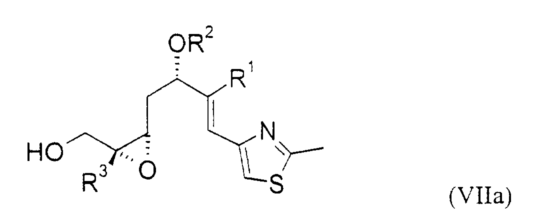 worin
R¹ Wasserstoff oder C₁-C₄-Alkyl und
R² p-Methoxybenzyl
R³ Wasserstoff oder C₁-C₄-Alkyl
bedeuten.

10. Verwendung der Verbindungen gemäß Ansprüche 1, 2, 3, 4, 5, 6, 7 und/oder 9 zur Herstellung von Epothilon A und Epothilon B und deren Derivaten.

## Claims

1. Compounds of the general formula II 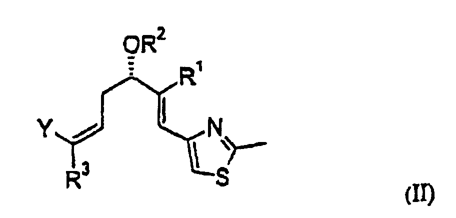 in which
R¹ is C₁-C₄-alkyl,
R² is any protective group capable of chelation,
R³ is hydrogen or C₁-C₄-alkyl,
Y is CO₂R⁴, CHO, CH=CH₂ or CH₂R⁵, where
R⁴ is C₁-C₄-alkyl or an optionally substituted benzyl group,
R⁵ is halogen, hydroxyl, p-toluene-sulphonate or -OSO₂B and
B is C₁-C₄-alkyl or C₁-C₄-perfluoroalkyl.

2. Compounds of the general formula II 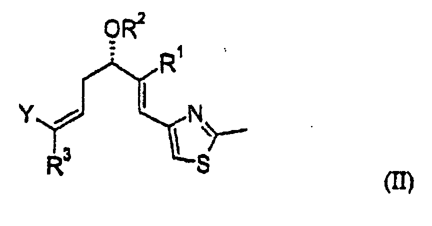 in which
R¹ is C₁-C₄-alkyl,
R² is p-methoxybenzyl,
R³ is methyl and
Y is CO₂R⁴
with R⁴ C₁-C₄-alkyl.

3. Compound of the formula IV 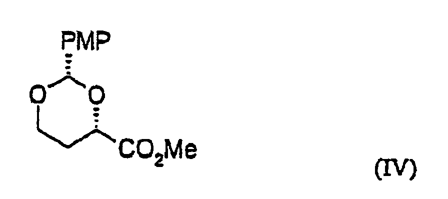 in which PMP is p-methoxyphenyl.

4. Compounds of the formula V 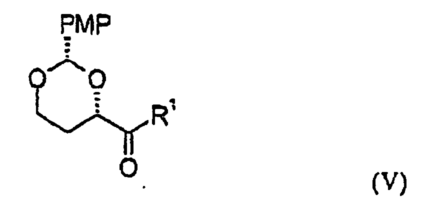 in which
R¹ is C₁-C₄-alkyl and
PMP is p-methoxyphenyl.

5. Compounds of the formula VI 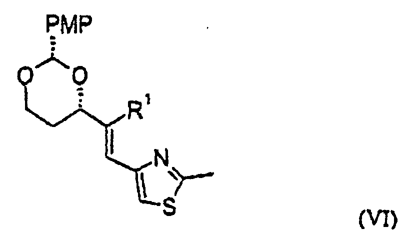 in which
R¹ is C₁-C₄-alkyl and
PMP is p-methoxyphenyl.

6. Compounds of the formula IIa 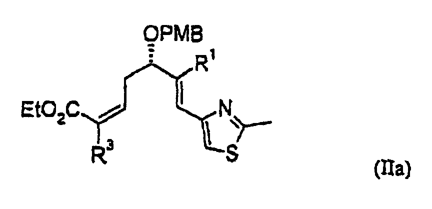 in which
R¹ is C₁-C₄-alkyl
PMB is p-methoxybenzyl,
R³ is hydrogen or C₁-C₄-alkyl.

7. Compounds of the formula VII 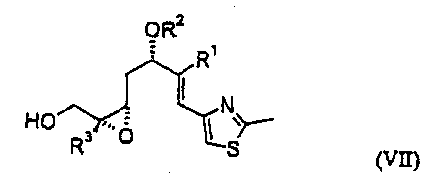 in which
R¹ is C₁-C₄-alkyl
R² is a protective group capable of chelation,
R³ is hydrogen or C₁-C₄-alkyl.

8. Process for the preparation of the compound of the general formula IIa 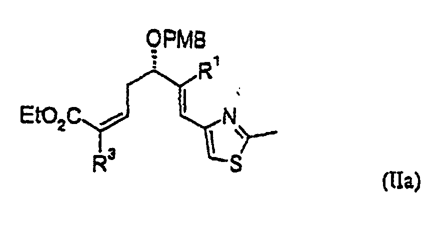 **characterized in that**
in a step 1
the α-hydroxy acid function of (S)-malic acid (III) is converted with trifluoroacetic acid/methanol (a) into the methyl ester, the acid function which is still present is reduced with diborane in tetrahydrofuran (b) to the alcohol, and the (S)-(-)-methyl-2,4-dihydroxy ester obtained in this way is converted with p-methoxybenzaldehyde dimethyl acetal (c) into the cyclic acetal (IV), 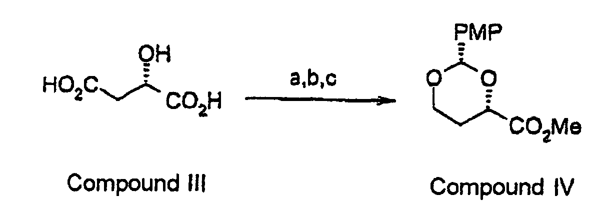 in a step II
the methyl ester is converted with a C₁-C₄-alkyl organometallic compound (d) into the corresponding alkyl ketone (V), 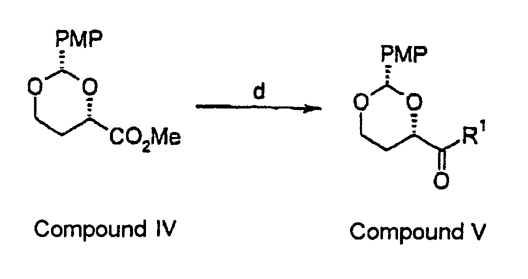 in a step III
the (C₁-C₄)-alkyl ketone (V) is reacted in a Wittig reaction with the thiazolylphosphonium salt (e) and the E isomer (VI) is separated out and 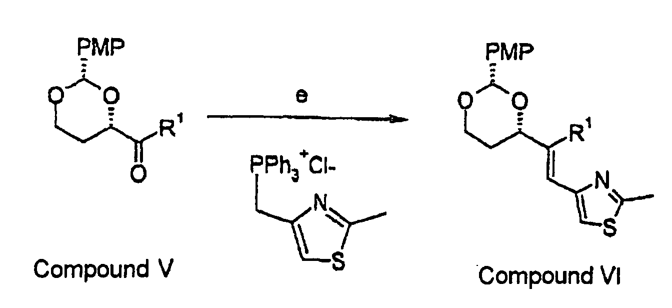 in a step IV
the E isomer (VI) is converted by reaction with diisobutylaluminium hydride (f), Swern oxidation (g) and Wadsworth-Horner-Emmons condensation (h) with ethyl 2-diethoxyphosphinylpropionate or a Horner reagent appropriate for R³, and purification from the E isomer, into the Z-α,β-unsaturated ester (IIa)

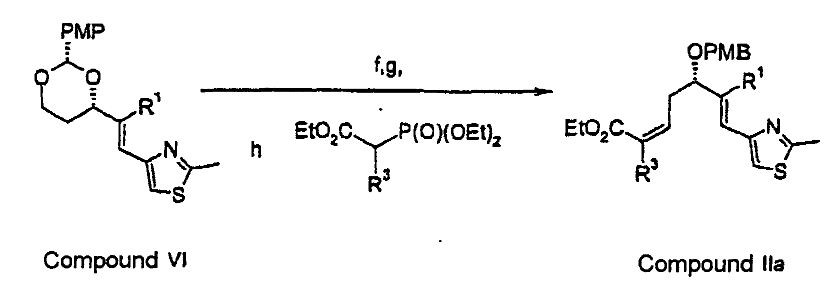

9. Compounds of the general formula VIIa 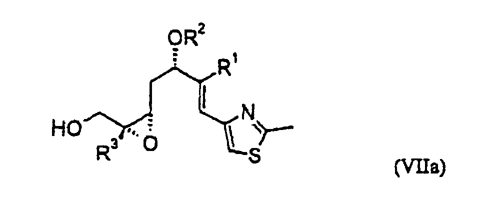 in which
R¹ is hydrogen or C₁-C₄-alkyl and
R² is p-methoxybenzyl
R³ is hydrogen or C₁-C₄-alkyl.

10. Use of the compounds according to Claims 1, 2, 3, 4, 5, 6, 7 and/or 9 for the preparation of epothilone A and epothilone B and derivatives thereof.

## Revendications

1. Composés de formule générale II 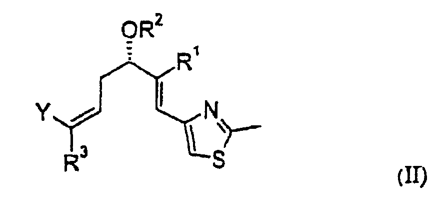 dans laquelle
R¹ représente un alkyle en C₁-C₄,
R² représente un groupe protecteur quelconque susceptible d'être chélaté,
R³ représente un hydrogène ou un alkyle en C₁-C₄,
Y représente CO₂R⁴, CHO, CH=CH₂ ou CH₂R⁵,
où
R⁴ représente un alkyle en C₁-C₄ ou un groupe benzyle éventuellement substitué,
R⁵ représente un halogène, un hydroxy, un p-toluènesulfonate ou -OSO₂B et
B représente un alkyle en C₁-C₄ ou un perfluoroalkyle en C₁-C₄.

2. Composés de formule générale II 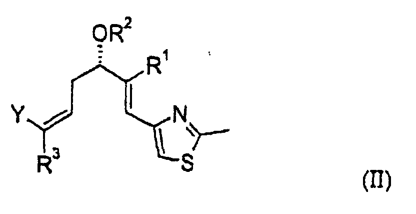 dans laquelle
R¹ représente un alkyle en C₁-C₄,
R² représente un p-méthoxybenzyle,
R³ représente un méthyle et
Y représente CO₂R⁴
où R⁴ représente un alkyle en C₁-C₄.

3. Composé de formule IV 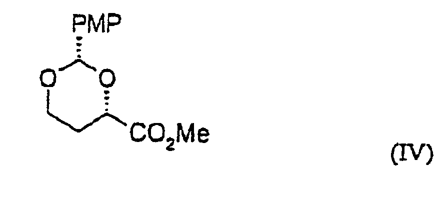 dans laquelle PMP représente un p-méthoxyphényle.

4. Composés de formule V 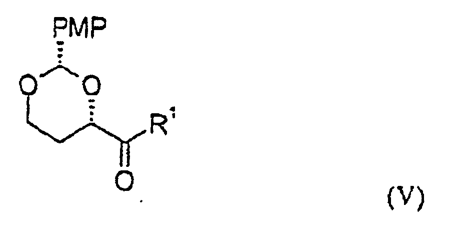 dans laquelle
R¹ représente un alkyle en C₁-C₄ et
PMP représente un p-méthoxyphényle.

5. Composés de formule VI 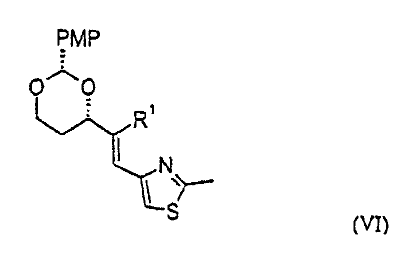 dans laquelle
R¹ représente un alkyle en C₁-C₄ et
PMP représente un P-méthoxyphényle.

6. Composés de formule IIa 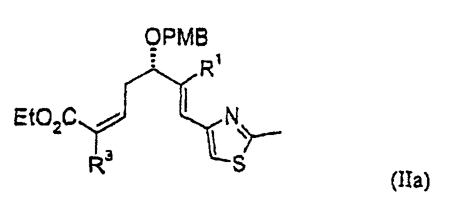 dans laquelle
R¹ représente un alkyle en C₁-C₄,
PMB représente un p-méthoxybenzyle,
R³ représente un hydrogène ou un alkyle en C₁-C₄.

7. Composés de formule VII 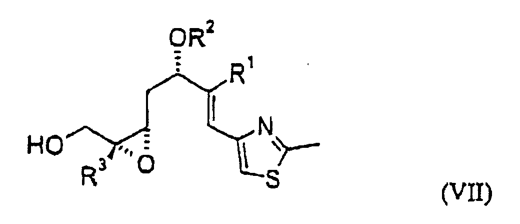 dans laquelle
R¹ représente un alkyle en C₁-C₄,
R² représente un groupe protecteur susceptible d'être chélaté,
R³ représente un hydrogène ou un alkyle en C₁-C₄.

8. Procédé de préparation du composé de formule générale IIa 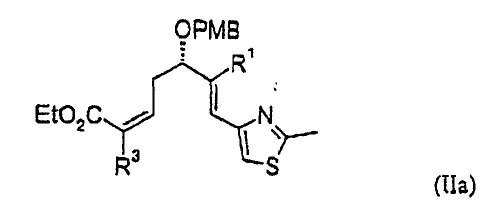 **caractérisé en ce que**
dans une étape I
à partir de l'acide (S)-malique (III), la fonction α-hydroxyacide est transformée avec de l'acide trifluoroacétique/méthanol (a) en l'ester méthylique, la fonction acide encore présente est réduite en alcool avec du diborane dans du tétrahydrofurane (b) et le (S)-(-)-méthyl-2,4-dihydroxyester ainsi obtenu est transformé avec du p-méthoxybenzyldiméthylacétal (c) en l'acétal cyclique (IV), 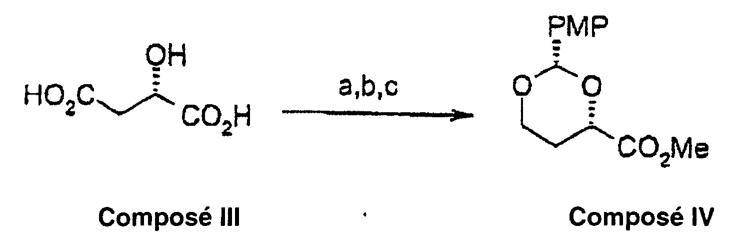 dans une étape II
l'ester méthylique est transformé avec un composé C₁-C₄-alkyl-organométallique (d) en l'alkylcétone (V) correspondante, 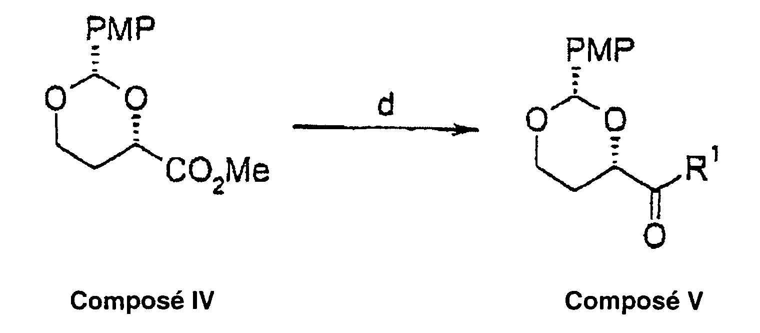 dans une étape III
la (C₁-C₄)-alkylcétone (V) est mise à réagir avec le sel de thiazolylphosphonium (e) dans une réaction de Wittig et l'isomère E (VI) est séparé, et 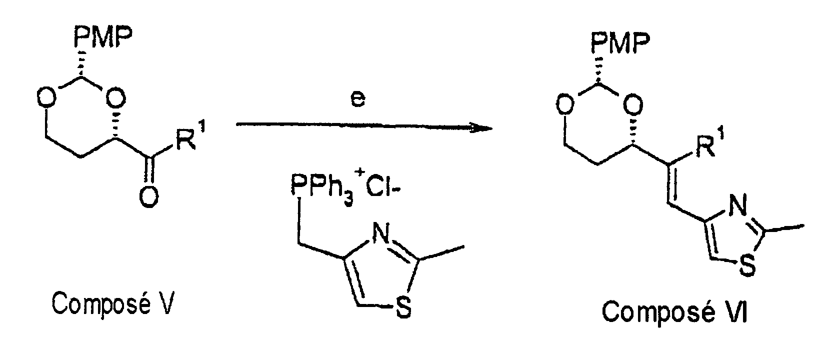 dans une étape IV
l'isomère E (VI) est transformé en l'ester Z-α,β-insaturé (IIa) par réaction avec de l'hydrure de diisobutylaluminium (f), oxydation de Swern (g) et condensation de Wadsworth-Horner-Emmons (h) avec du 2-diéthoxyphosphinylpropionate d'éthyle ou un réactif de Horner correspondant pour R³ et purification de l'isomère E.

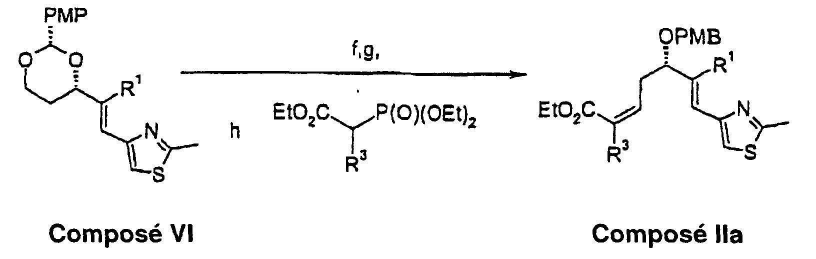

9. Composés de formule générale VIIa 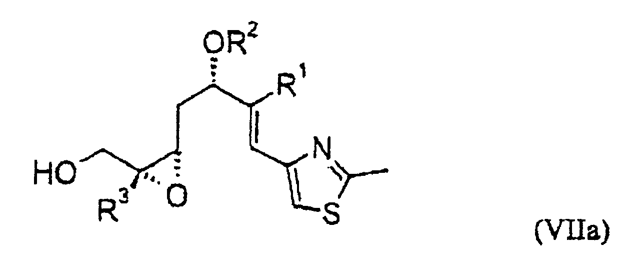 dans laquelle
R¹ représente un hydrogène ou un alkyle en C₁-C₄ et
R² représente un p-méthoxybenzyle,
R³ représente un hydrogène ou un alkyle en C₁-C₄.

10. Utilisation des composés selon les revendications 1, 2, 3, 4, 5, 6, 7 et/ou 9 pour la préparation de l'épothilone A et de l'épothilone B et de leurs dérivés.
